# EUROPÄISCHE PATENTSCHRIFT

(11) **EP 0 884 033 B1**
(45) Veröffentlichungstag und Bekanntmachung des Hinweises auf die Patenterteilung: **25.09.2002**
(21) Anmeldenummer: 98106689.7
(22) Anmeldetag: 11.04.1998
(51) Int. Cl.: A61F 2/66

(54) **Fusseinsatz für einen Kunstfuss**
Insert for artificial foot
Insert pour pied artificiel

(30) Priorität: 24.04.1997 DE 29707416 U
(43) Veröffentlichungstag der Anmeldung: 16.12.1998
(73) Patentinhaber: Otto Bock HealthCare GmbH, 37115 Duderstadt (DE)
(72) Erfinder: Mosler, Lüder, Dr., 37115 Duderstadt (DE); Pusch, Martin, 37115 Duderstadt (DE)
(74) Vertreter: Gramm, Werner, Prof. Dipl.-Ing.

(56) Entgegenhaltungen:
- EP-A- 0 444 883
- DE-A- 4 205 900
- DE-C- 363 938
- FR-E- 25 322
- US-A- 5 139 525
- US-A- 5 387 246

## Beschreibung

Die Erfindung betrifft einen federelastischen Fußeinsatz für einen Kunstfuß, mit einer als Blattfeder ausgebildeten Vorfußfeder und einer im Fersenbereich angeordneten, ebenfalls als Blattfeder ausgebildeten Fersenfeder, wobei zumindest eines dieser beiden Federelemente die Federsteifigkeit des Kunstfußes bestimmt, und mit einer Adaptierungseinrichtung zur Veränderung der Federsteifigkeit des zumindest einen die Federsteifigkeit des Kunstfußes bestimmenden Federelementes.

Eine derartige Ausführungsform lässt sich der DE-A-42 05 900 entnehmen. Der Fußeinsatz besteht hier aus drei Blattfedern, von denen eine flach gebogene Blattfeder im Fersenbereich angeordnet und mit ihrem vorderen Ende im Vorfußbereich mit einer gebogenen Vorfußfeder verbunden ist, die sich mit ihrem einen Ende nach oben in den Fußanschlussbereich erstreckt und hier mit einem Adapter verschraubt ist, während das andere Federende bis in den vorderen Fußbereich ragt. Dieser Vorfußfeder ist eine zweite, etwas kürzer ausgebildete Blattfeder zugeordnet, die an ihrem oberen Ende im Bereich des Adapteranschlusses mit dem oberen Ende der Vorfußfeder verschraubt ist und mit letzterer einen keilförmig sich nach vorn vergrößernden Aufnahmeraum begrenzt, in den ein luftgefülltes Druckkissen angeordnet ist, das sich mit seiner Ober- und Unterseite an den beiden genannten Blattfedern abstützt. Der Patient kann die Federspannungsdaten dieses Fußeinsatzes durch Veränderung des Luftdrucks im Druckkissen verändern. Mit Hilfe einer manuell zu betätigenden Handpumpe lässt sich das Druckkissen aufblasen; eine Ventileinrichtung erlaubt ein Abblasen des Druckkissen-Innendruckes.

Die DE-A-363 938 offenbart einen starr ausgebildeten Kunstfuß, dessen Fersenteil aus einer Luftkammer aus Gummi besteht und dessen Zehenteil als Luftkammer ausgebildet ist, wobei diese beiden Kammern durch Kanäle derart miteinander in Verbindung stehen, dass die Luft bei Belastung der einen Kammer in die andere übertreten kann. Das Körpergewicht wird von dem starren mittleren Fußteil aufgefangen und auf eine abgerundete Abwälzfläche übertragen. Beim Aufsetzen der Fußspitze wird die Luft durch die genannten Kanäle in die den Fersenteil bildende Kammer gedrängt, während sich beim Aufsetzen der Ferse der umgekehrte Vorgang abspielt. Zur geregelten Durchführung dieses Ausgleiches ist die Luft in beiden Kammern so verdichtet, dass eine genügende Nachgiebigkeit entsprechend dem Körpergewicht gesichert ist.

Der Erfindung liegt die Aufgabe zugrunde, den Tragekomfort des eingangs beschriebenen Fußeinsatzes zu verbessern.

Diese Aufgabe wird erfindungsgemäß dadurch gelöst, dass die Beaufschlagung der Adaptierungseinrichtung durch eine sich in Abhängigkeit von der jeweiligen Belastung des Fußeinsatzes durch den den Kunstfuß tragenden Patienten einstellende Fersenauslenkung im Fußeinsatz erfolgt.

In einer bevorzugten Ausführungsform ist es zweckmäßig, wenn das hinsichtlich seiner Federsteifigkeit zu verändernde Federelement die Vorfußfeder ist, die sich nahezu über die gesamte Länge des Fußeinsatzes erstreckt, und dass die sich durch die Fersenbelastung einstellende Fersenauslenkung im Fußeinsatz als Eingangsgröße für eine Zwangsregelung der Adaptierungseinrichtung dient.

Erfindungsgemäß wird somit die Steifigkeitseinstellung des Fußeinsatzes geregelt als Funktion der Gangdynamik des Patienten. Da die Federwege der Ferse in der Regel deutlich kleiner eingestellt sind als die des Vorfußes, ist der Einfluß des Fersenweges auf das Gangbild geringer. Erfindungsgemäß wird daher die Fersenauslenkung zur Steuerung der Vorfußsteifigkeit verwendet.

In einer weiteren Ausführungsform ist es vorteilhaft, wenn die Vorfußfeder von einem ihre Federsteifigkeit bestimmenden luftgefüllten Druckkissen beaufschlagt wird, und die Adaptierungseinrichtung eine Luftpumpe ist, die durch die Fersenauslenkung angetrieben wird und somit bei steigender Fersenbelastung den Luftdruck im Druckkissen erhöht, während bei abnehmender Fersenbelastung der Luftdruck im Druckkissen abnimmt.

Dabei kann es wegen der nichtlinearen Kraft-Weg-Charakteristik einer Luftfeder vorteilhaft sein, wenn dem luftgefüllten Druckkissen eine konturierte Anlagefläche zugeordnet ist, deren auf das Druckkissen projizierte Fläche beim Eindringen in das Druckkissen so auf den Verlauf des Druckanstiegs infolge der Volumenabnahme im Druckkissen angepasst ist, dass sich eine zumindest angenähert lineare Kraft-Weg-Charakteristik des Druckkissens ergibt. Eine derartige Linearisierung lässt sich auch durch eine degressive Annäherung der beiden das Druckkissen zwischen sich beaufschlagenden Anlageflächen erreichen.

Die Gangdynamik des Patienten ist eine Funktion seiner Tagesform aber auch seiner aktuellen Beschäftigung. Während die Funktion einer Anpassung an die Tagesform durchaus durch ein sukzessive anpassendes System (wie vorstehend beschrieben mit Hilfe eines Luftkissens) denkbar ist, muss die Anpassung an die momentane Beschäftigung (etwa am Arbeitsplatz) teilweise sehr schnell, praktisch von einem Schritt zum nächsten erfolgen. Eine derart schnelle Anpassung kann jedoch ein pneumatisches System kaum leisten.

Um eine schnelle, automatische Anpassung des Prothesenfußes an die aktuelle Gangdynamik des Patienten zu verwirklichen, wird erfindungsgemäß vorgeschlagen, dass die Adaptierungseinrichtung ein starrer, in Längsrichtung gegenüber der Vorfußfeder verschiebbarer und deren Federsteifigkeit bestimmender Abstandshalter ist, dessen Längsverschiebung durch eine mechanische Verstelleinrichtung erfolgt, die kinematisch mit der Fersenauslenkung verbunden ist, die bei maximaler Auslenkung eine maximale Verschiebung des Abstandshalters in Richtung des vorderen Endes der Vorfußfeder bewirkt, während eine Vorfußentlastung eine Rückbewegung des Abstandshalters in seine Ausgangsposition hervorruft.

Tritt bei dieser Ausführungsform der Patient bei einem zügigen Schritt fest auf die Ferse, wird der Abstandshalter weiter nach vorne verschoben; bei der darauf folgenden Belastung des Vorfußes wird die Position des Abstandshalters fixiert. Der Patient kann dann mittels des steiferen Vorfußhebels einen längeren Schritt ausführen als ihm dies bei einem weicheren Vorfußhebel möglich wäre. Nach Entlastung des Vorfußes entfällt diese Fixierung, und der Abstandshalter kann in seine Ausgangsposition zurückkehren. Eine derartige Anordnung erlaubt die Adaptierung der Vorfußfederhärte für jeden einzelnen Schritt, was bei stark variierenden Tätigkeiten von Vorteil ist.

Die in derartigen Fußeinsätzen verwendeten Blattfedern sind einer außerordentlich hohen Belastung ausgesetzt. Dabei können die verwendeten Federn aus Carbonkomposit, aus Titan oder auch aus anderen geeigneten Materialien gefertigt sein. Die funktionell erforderliche Verformung führt zu hohen Spannungen, zu deren Aufnahme die Dauerfestigkeit der verwendeten Blattfedern häufig nicht ausreicht. Um die Strukturfestigkeit der in federelastischen Fußeinsätzen verwendeten Blattfedern zu erhöhen, wird erfindungsgemäß vorgeschlagen, daß zumindest die Vorfuß- oder Basisfeder aus zwei parallel geschalteten Blattfederelementen besteht, die mit ihrem hinteren Ende momentenstarr gekoppelt sind. Während eine bloße Materialverstärkung das Problem in der Regel nicht zu lösen vermag, läßt sich die Strukturfestigkeit durch Parallelschaltung von zwei weicheren Federn überraschend wirksam verbessern, ohne hierdurch die erforderliche Federcharakteristik in unerwünschter Weise zu verändern. Dabei läßt sich das Ergebnis in einigen Fällen noch dadurch verbessern, daß die beiden parallel geschalteten Blattfederelemente in ihren beiden Endbereichen momentenstarr gekoppelt sind.

Weitere Merkmale der Erfindung sind Gegenstand der Unteransprüche und werden in Verbindung mit weiteren Vorteilen der Erfindung anhand von Ausführungsbeispielen näher erläutert.

In der Zeichnung sind einige als Beispiele dienende Ausführungsformen der Erfindung dargestellt. Es zeigen:
- **Figur 1**: in Seitenansicht ein sich pneumatisch selbst adaptierender elastischer Fußeinsatz;
- **Figur 2**: in einer Darstellung gemäß Figur 1 eine abgewandelte Ausführungsform eines sich mechanisch selbst adaptierenden Kunstfußes mit längsverschieblich angeordnetem Abstandshalter;
- **Figur 3**: eine abgewandelte Ausführungsform in einer Darstellung gemäß Figur 2;
- **Figur 4**: im Längsschnitt in der Sagitalebene eine Vorfußfeder mit federelastischen Druckpuffern;
- **Figur 5**: eine abgewandelte Ausführungsform in einer Darstellung gemäß Figur 1;
- **Figur 6**: in schematischer Darstellung ein Detail der Figur 5;
- **Figur 7**: eine abgewandelte Ausführungsform in einer Darstellung gemäß Figur 2;
- **Figur 8**: eine abgewandelte Ausführungsform in einer Darstellung gemäß Figur 1 und
- **Figur 9**: eine weiterhin abgewandelte Ausführungsform in einer Darstellung gemäß Figur 1.

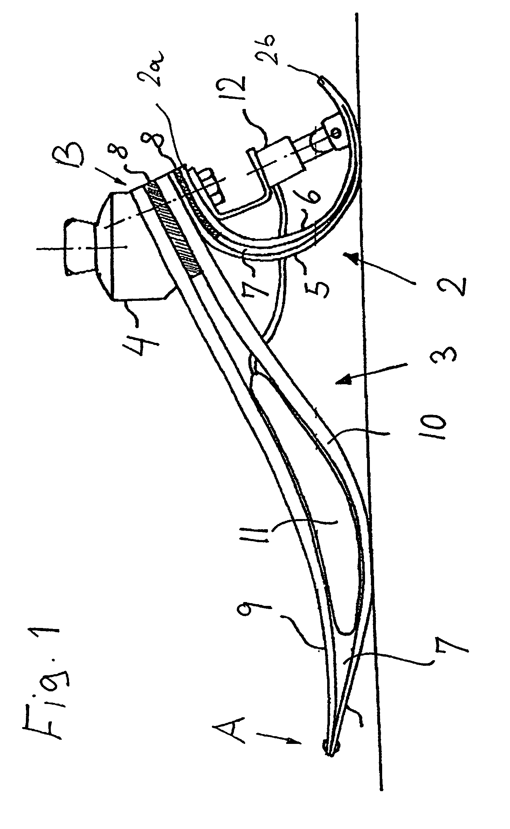

Figur 1 zeigt einen federelastischen Fußeinsatz, der eine C-förmig ausgebildete Fersenfeder 2 aufweist, die mit ihrem oberen Schenkel 2a mit dem hinteren Endbereich B einer Vorfußfeder 3 verschraubt ist. Der obere Schenkel 2a der C-Feder 2 ist mit einem Adapter 4 verschraubt, über den der Kunstfuß an eine Beinprothese anschließbar ist.

Die C-Feder 2 besteht aus zwei parallel geschalteten Blattfederelementen 5, 6, die nebeneinander angeordnet sind, angenähert parallel zueinander verlaufen, in ihren beiden Endbereichen miteinander verbunden sind und zwischen diesen beiden Endbereichen einen lichten Abstand 7 voneinander aufweisen. In diesen beiden Endbereichen ist zwischen den beiden Blattfederelementen 5, 6 jeweils ein Distanzelement 8 vorgesehen.

Die Vorfußfeder 3 setzt sich ebenfalls zusammen aus zwei parallel geschalteten Blattfederelemente 9, 10, die nebeneinander angeordnet und in ihren beiden Endbereichen A, B momentenstarr miteinander verbunden sind. Dabei ist nur im hinteren Endbereich B zwischen den beiden Blattfederelementen 9, 10 ein Distanzelement 8 vorgesehen. Zwischen den beiden Endbereichen A, B weisen die beiden Blattfederelemente 9, 10 einen lichten Abstand 7 voneinander auf, in den ein luftgefülltes Druckkissen 11 eingesetzt ist, das an den beiden Blattfederelementen 9, 10 spannungsfrei anliegt.

Das luftbefüllte Druckkissen 11 ist an den Druckstutzen eines Luftpumpenelementes 12 angeschlossen, das in dem von der C-Feder 2 umschlossenen Raum angeordnet und in Abhängigkeit vom Patientengewicht- und/oder durch die Patientenaktivität betätigbar ist. Die C-Feder 2 wird unter Fersenbelastung etwas zusammengedrückt, wobei die sich dadurch einstellende Abstandsänderung der beiden C-Federschenkel 2a, 2b voneinander eine Fersenauslenkung definiert, die den Antrieb für das Luftpumpenelement 12 darstellt.

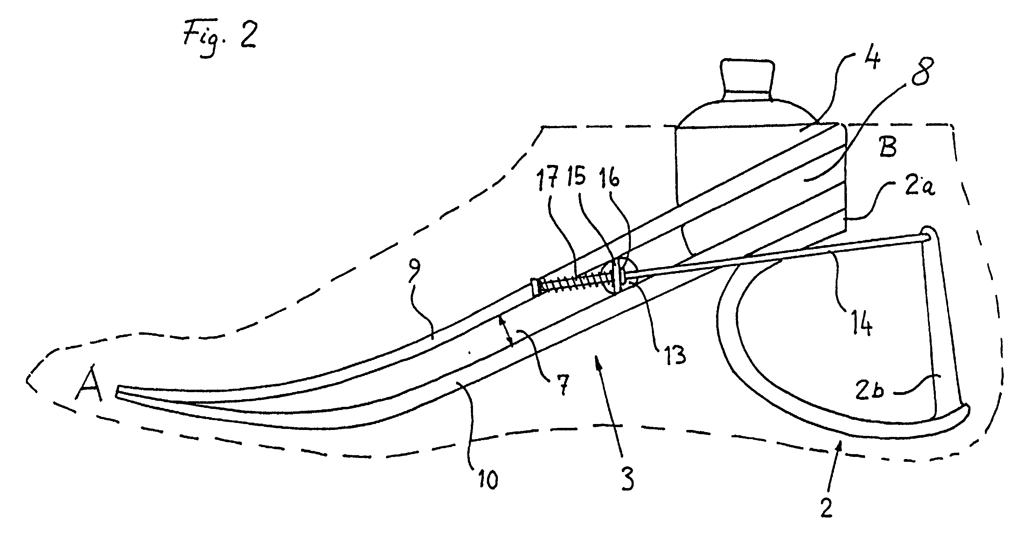

Bei der Ausführungsform gemäß Figur 2 entsprechen die C-Feder 2 und die Vorfußfeder 3 im wesentlichen denen der Figur 1. Jedoch ist anstelle einer pneumatischen Adaptierung in Figur 2 eine mechanische Adaptierung dargestellt. Hier ist das untere freie Schenkelende der C-Feder 2 über eine Schubstange 14 mit einem starren Abstandshalter 13 kinematisch verbunden. Das nach vorn weisende Ende der Schubstange 14 ist längsverschieblich in einer seitlich am Abstandshalter 13 sitzenden Kulisse 15 geführt, liegt rückseitig mit einem Mitnehmer 16 an der Kulisse 15 an und stützt sich mit ihrem freien Ende über eine Feder 17 an der Kulisse 15 ab. Tritt der Patient bei einem zügigen Schritt fest auf die Ferse, führt die Verformung der C-Feder 2 (Fersenauslenkung) zu einer Verschiebung der Schubstange 14, die über ihren Mitnehmer 16 den Abstandshalter 13 weiter nach vorn verschiebt, wo er bei der darauf folgenden Belastung des Vorfußes fixiert wird. Die Feder 17 ermöglicht ein Zurückfedern der Fersenfeder, ohne hierbei die Position des Abstandshalters 13 zu verändern. Nach Entlastung des Vorfußes entfällt die Fixierung des Abstandshalters 13, der daraufhin unter Einwirkung der schwach ausgelegten Feder 17 wieder in seine Ausgangsposition zurückkehrt.

Bei dem Lösungsvorschlag gemäß Figur 2 wird somit eine schnelle, automatische Anpassung des Prothesenfußes an die aktuelle Gangdynamik des Patienten verwirklicht, wobei die Steifigkeit der zweilagigen Vorfußfeder 3 mit einem längsverschieblichen, mechanischen Abstandshalter 13 eingestellt wird, dessen Steuerung über die Härte des Fersenauftritts erfolgt.

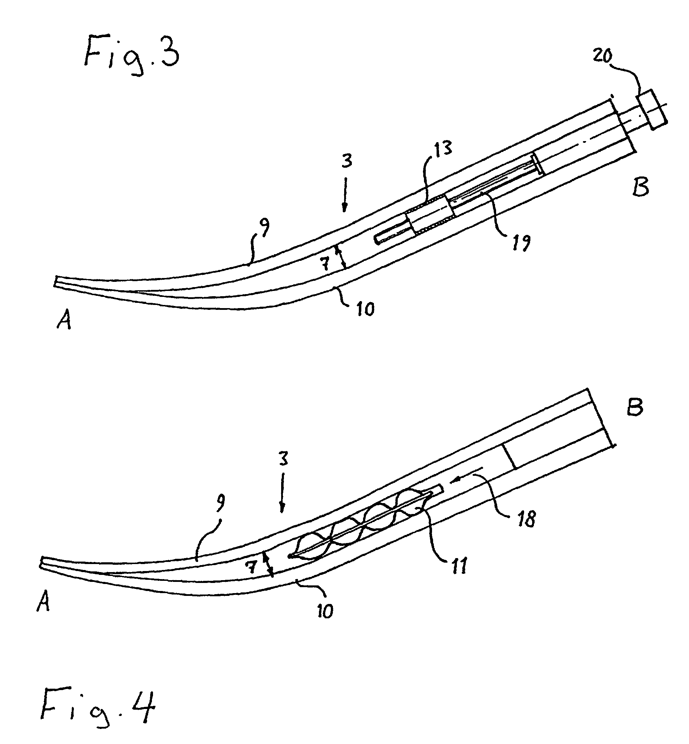

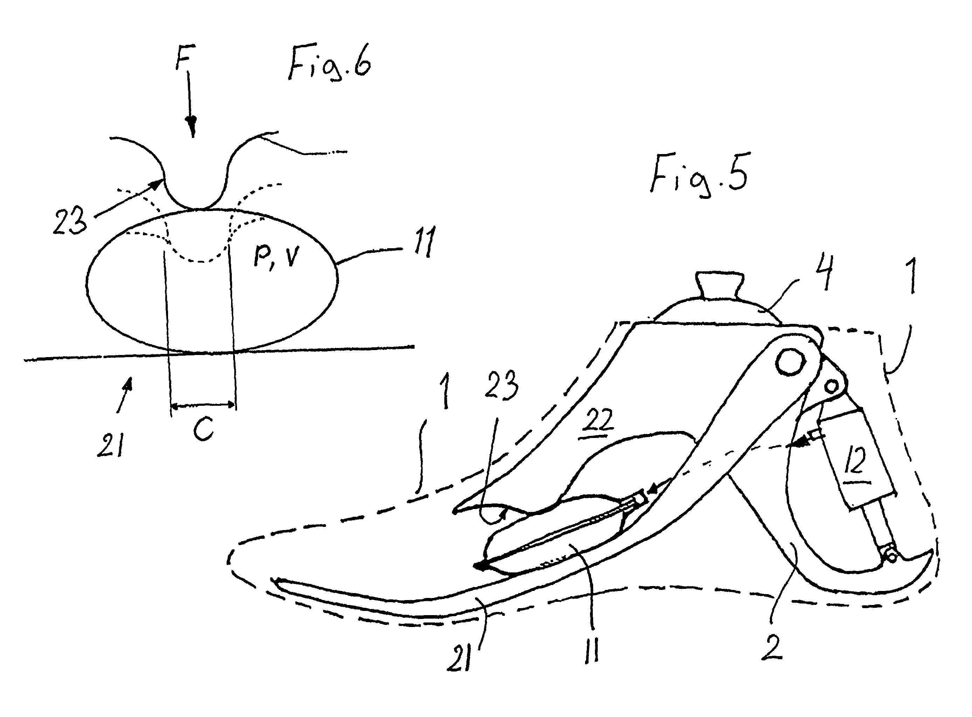

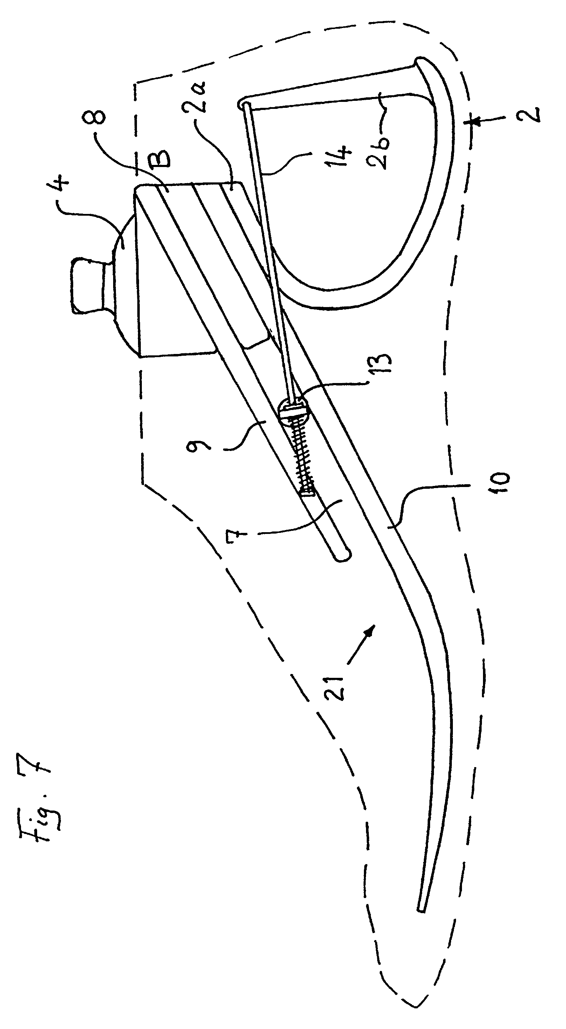

Figur 3 zeigt eine mit Figur 2 vergleichbare Vorfußfeder 3, deren starre Abstandshalter 13 über eine Spindel 19 längsverschieblich ist, auf der der Abstandshalter 13 mit einer nicht näher dargestellten Spindelmutter geführt ist. Die Spindel 19 ragt mit ihrem hinteren Ende aus dem hinteren Endbereich B der Vorfußfeder 3 heraus und wird hier angeschlossen an eine nicht näher dargestellte mechanische Verstelleinrichtung, die kinematisch mit der Fersenauslenkung der C-Feder verbunden ist.

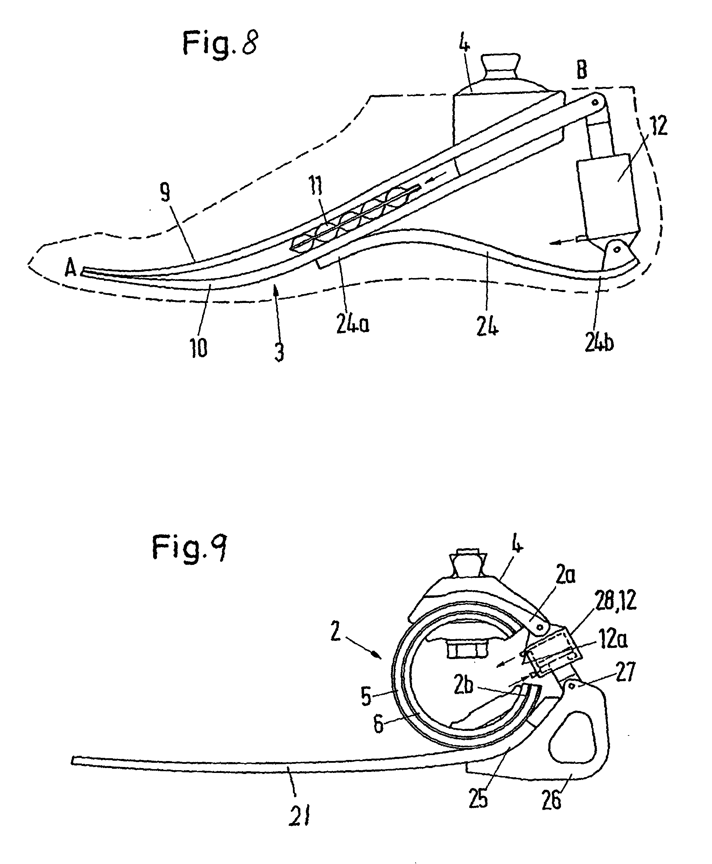

Figur 4 zeigt eine Vorfußfeder 3, die in dem lichten Abstand 7 zwischen ihren beiden Blattfederelementen 9, 10 ein elastisches Druckkissen 11 aufnimmt, das - wie es der Pfeil 18 andeutet - entweder in Längsrichtung der Vorfußfeder 3 über eine mechanische Einrichtung z. B. gemäß Figur 2 oder Figur 3 in Abhängigkeit von der Fersenbelastung verschiebbar oder aber in der dargestellten Position mit einer fersenbelastungsabhängigen Steuerung z. B. gemäß Figur 1 aufblasbar ist.

Der in Figur 5 dargestellte gelenklose Kunstfuß weist eine strichpunktiert angedeutete kosmetische Hülle 1 auf, die einen federelastischen Fußeinsatz umschließt. Letzerer entspricht hinsichtlich der Beaufschlagung des luftgefüllten Druckkissens 11 der Ausführungsform gemäß Figur 1. Jedoch liegt das Druckkissen 11 gemäß Figur 5 auf einer verschwenkbaren, ebenfalls als Blattfeder ausgebildeten, bis in den Vorfuß reichenden Basisfeder 21 auf, die mit ihrem hinteren Ende im oberen Bereich der C-Feder 2 verschwenkbar angelenkt ist. Das Druckkissen 11 wird auf seiner Oberseite von einem steif ausgebildeten Schenkel 22 des Fußeinsatzes beaufschlagt. Die Anlagefläche 23 dieses steifen Schenkels 22 ist konturiert ausgebildet.

Figur 6 soll verdeutlichen, daß die auf das Druckkissen 11 projizierte Fläche C der konturierten Anlagefläche 23 beim Eindringen in das Druckkissen 11 so auf den Verlauf des Druckanstiegs infolge der Volumenabnahme im Druckkissen 11 angepaßt ist, daß sich eine zumindest angenähert lineare Kraft-Weg-Charakteristik des Druckkissens 11 ergibt. Dabei ist die Fläche C in der eingezeichneten Kraftrichtung F projiziert.

Da Luftfedern in der Regel eine nichtlineare Kraft-Weg-Charakteristik besitzen wird erfindungsgemäß die Anlagefläche 23 so konturiert, daß linearisierte Kennlinien entstehen. Das Kraft-Weg-Verhalten derart luftgefüllter Druckkissen ist im isothermen Fall eine Funktion der anliegenden, in Kraftrichtung projizierten Fläche C sowie des Kehrwertes des Restvolumens V im Druckkissen, dessen Innendruck P sich dann aus dem Quotienten F/C ergibt.

Die Ausführungsform gemäß Figur 7 entspricht hinsichtlich des Abstandshalters und seiner kinematischen Verbindung mit der C-Feder dem Lösungsvorschlag gemäß Figur 2, jedoch sind hier die beiden die Vorfußfeder 3 bildenden Blattfederelemente 9, 10 nur an ihrem hinteren Ende B momentenstarr miteinander gekoppelt.

Der in Figur 8 dargestellte Fußeinsatz ist gekennzeichnet durch eine sich über nahezu die gesamte Länge des Fußeinsatzes erstreckende Vorfußfeder 3 und eine flach sinusförmig ausgebildete Fersenfeder 24, die mit ihrem vorderen Schenkelende 24a im mittleren Längsbereich der Vorfußfeder 3 an deren Unterseite befestigt ist. Die Adaptierungseinrichtung ist wiederum durch ein Luftpumpenelement 12 gebildet, das zwischen dem hinteren Schenkelende 24b der Fersenfeder 24 und dem hinteren Ende der Vorfußfeder 3 angeordnet ist. Es handelt sich also wiederum um eine pneumatisch adaptierende Ausführung, deren Vorzug darin zu sehen ist, daß eine größere Durchbiegung des Vorfußes für einen größeren Gesamtweg der Adaptiereinrichtung sorgt. Bei Verwendung einer Luftpumpe als Adaptiereinrichtung wird somit mehr Luft angesaugt bzw. mehr Luft in die luftgefüllten Druckkissen 11 und somit in den Vorfuß gefördert. Bei erfolgreicher Adaptierung läßt die Durchbiegung des Vorfußes aufgrund der größeren Federsteifigkeit nach, so daß die Pumpe wieder das als üblich festgelegte Volumen fördert.

Der in Figur 9 dargestellte Fußeinsatz besteht aus einer sich über nahezu die gesamte Länge des Fußeinsatzes erstreckenden Basisfeder 21 sowie aus einer im Fersenbereich angeordneten C-Feder 2, die mit ihrem oberen C-Schenkel 2a die Prothesenbelastung aufnimmt und mit ihrem unteren C-Schenkel 2b auf einem von dem hinteren Ende der Basisfeder 21 gebildeten nach hinten hochgezogenen Sattel 25 aufliegt. Letzterer stützt sich auf einem Fersenkeil 26 ab. Zwischen dem oberen C-Schenkel 2a und einem hinter ihm angeordneten Lagerbock 27 ist eine Fesselung in Form eines nicht näher dargestellten Pneumatikzylinders 28 vorgesehen, der eine progressive Kraft-Weg-Kennlinie aufweist. Außerdem ist zwischen dem oberen C-Schenkel 2a und dem Lagerbock 27 eine Luftpumpe 12 angelenkt, deren Druckstutzen 12a an den Pneumatikzylinder 28 angeschlossen ist.

Der Pneumatikzylinder 28 dient somit als Fesselglied zur Veränderung der Federeigenschaften des Fußeinsatzes. Eine Vorfußbelastung führt über das von ihr ausgehende Moment zu einer Aufweitung der C-Feder 2. Die hierbei auftretenden Kräfte sollen über das Fesselglied in die Basisbefestigung abgeleitet werden. Das Fesselglied soll aber in beiden Richtungen der Verformung der C-Feder 2, also sowohl beim Zubiegen als auch beim Aufbiegen wirken. Dabei soll das Zubiegen der C-Feder am Anfang möglichst weich erfolgen, während bei weiterer Verformung die vom Fesselglied auf die Verformung der C-Feder ausgeübte begrenzende Wirkung zunehmen soll. Diese Progression stellt sich für den Patienten durch ein erleichtertes Abrollen auf den Vorfuß dar, da eine durch eine derartige Fesselung unterstützte C-Feder weicher ausgeführt werden kann. Dabei schlägt sich der für den Patienten spürbar geringere Vorfußwiderstand auch in dem Knöchelmomentenverlauf in der Standphase nieder. Um nun diese Progression automatisch in Abhängigkeit von der jeweiligen Belastung des Fußeinsatzes durch den den Kunstfuß tragenden Patienten aktiv zu steuern, ist als Adaption die dem Pneumatikzylinder parallel geschaltete Luftpumpe 12 vorgesehen, die mit ihrem Druckstutzen an den Pneumatikzylinder 28 angeschlossen ist. Letzterer ist in Figur 9 nicht dargestellt; er liegt hinter der Luftpumpe 12. Dabei dürfte es allerdings zweckmäßig sein, den Querschnitt der Luftpumpe 12 kleiner zu halten als den Querschnitt des Pneumatikzylinders. Drückt die Luftpumpe 12 eine zusätzliche Luftmenge in den Pneumatikzylinder 28, wird dessen Grunddruck und damit dessen Steifigkeit erhöht. Der Druckabbau im Pneumatikzylinder kann wiederum über eine Leckage erfolgen.

Die hintere, am Lagerbock 27 vorgesehene Fessellagerung liegt vorzugsweise im Achillessehnenbereich. Die vordere bzw. obere Fessellagerung kann unmittelbar am Adapter 4 angreifen.

Zusammenfassend wird somit für den Lösungsvorschlag gemäß Figur 9 festgestellt: Die Fesselung steuert die Aufbiegungsbegrenzung der C-Feder 2 in Abhängigkeit von der vorhergehenden Zubiegung der C-Feder 2, die der Fersenbelastung entspricht. Da die Vorfußsteifigkeit durch die Serienschaltung von C- und Basisfeder 2, 21 bestimmt wird, ergibt sich eine progressive Kennlinie, die durch die progressive Wegbegrenzung der Fesselung gesteuert wird. Dabei besteht der Vorzug dieser Lösung darin, daß durch Einflußnahme auf die Fesselungscharakteristik die Vorfußsteifigkeit sowie deren Kennlinie beeinflußt werden kann. Eine weichere, progressive Kennlinie erleichtert das Abrollen des Fußes, erlaubt aber nur eine eingeschränkte Gangdynamik und Energierückgabe. Eine härtere, lineare Kennlinie erlaubt hingegen eine höhere Gangdynamik, ist aber bei niedrigen Gehgeschwindigkeiten unbequem.

## Patentansprüche

1. Federelastischer Fußeinsatz für einen Kunstfuß, mit einer als Blattfeder ausgebildeten Vorfußfeder (3; 9, 10; 21) und einer im Fersenbereich angeordneten, ebenfalls als Blattfeder ausgebildeten Fersenfeder (2; 24), wobei zumindest eines dieser beiden Federelemente die Federsteifigkeit des Kunstfußes bestimmt, und mit einer Adaptierungseinrichtung zur Veränderung der Federsteifigkeit des zumindest einen die Federsteifigkeit des Kunstfußes bestimmenden Federelementes, **dadurch gekennzeichnet, dass** die Beaufschlagung der Adaptierungseinrichtung durch eine sich in Abhängigkeit von der jeweiligen Belastung des Fußeinsatzes durch den den Kunstfuß tragenden Patienten einstellende Fersenauslenkung im Fußeinsatz erfolgt.

2. Fußeinsatz nach Anspruch 1, **dadurch gekennzeichnet, dass** das hinsichtlich seiner Federsteifigkeit zu verändernde Federelement die Vorfußfeder (3) ist, die sich nahezu über die gesamte Länge des Fußeinsatzes erstreckt, und dass die sich durch die Fersenbelastung einstellende Fersenauslenkung im Fußeinsatz als Eingangsgröße für eine Zwangsregelung der Adaptierungseinrichtung dient.

3. Fußeinsatz nach Anspruch 2, **dadurch gekennzeichnet, dass** die Vorfußfeder (3) von einem ihre Federsteifigkeit bestimmenden luftgefüllten Druckkissen (11) beaufschlagt wird, und die Adaptierungseinrichtung eine Luftpumpe (12) ist, die durch die Fersenauslenkung angetrieben wird und somit bei steigender Fersenbelastung den Luftdruck im Druckkissen (11) erhöht, während bei abnehmender Fersenbelastung der Luftdruck im Druckkissen (11) abnimmt.

4. Fußeinsatz nach Anspruch 3, **gekennzeichnet durch** ein dem Druckkissen (11) zugeordnetes, eine konstante Überdruckleckage bildendes Überdruckventil zum Abbau des Luftdruckes innerhalb einer vorgegebenen Zeitspanne.

5. Fußeinsatz nach Anspruch 3, **dadurch gekennzeichnet, dass** die Adaptierungseinrichtung einen Zwischenspeicher umfasst, der den aktuellen Wert der Fersenauslenkung memoriert, wobei bei zunehmender Fersenauslenkung weitere Luft in das Druckkissen (11) gepumpt, bei abnehmender Fersenauslenkung der Luftdruck im Druckkissen (11) aber verringert wird.

6. Fußeinsatz nach Anspruch 3, 4 oder 5, **dadurch kennzeichnet,** dass dem luftgefüllten Druckkissen (11) eine konturierte Anlagefläche (23) zugeordnet ist, deren auf das Druckkissen (11) projizierte Fläche (C) beim Eindringen in das Druckkissen (11) so auf den Verlauf des Druckanstiegs infolge der Volumenabnahme im Druckkissen (11) angepasst ist, dass sich eine zumindest angenähert lineare Kraft-Weg-Charakteristik des Druckkissens (11) ergibt.

7. Fußeinsatz nach einem der Ansprüche 3 bis 6, **dadurch gekennzeichnet, dass** das luftgefüllte Druckkissen (11) auf einer verschwenkbaren, bis in den Vorfuß reichenden Vorfußfeder (21) aufliegt und auf seiner Oberseite von einem steif ausgebildeten Schenkel (22) des Fußeinsatzes beaufschlagt ist.

8. Fußeinsatz nach Anspruch 1 oder 2, **dadurch gekennzeichnet, dass** die Adaptierungseinrichtung ein starrer, in Längsrichtung gegenüber der Vorfußfeder (3; 21) verschiebbarer und deren Federsteifigkeit bestimmender Abstandshalter (13) ist, dessen Längsverschiebung durch eine mechanische Verstelleinrichtung (14, 15, 16, 17; 19, 20) erfolgt, die kinematisch mit der Fersenauslenkung verbunden ist, die bei maximaler Auslenkung eine maximale Verschiebung des Abstandshalters (13) in Richtung des vorderen Endes der Vorfußfeder (3; 21) bewirkt, während eine Vorfußentlastung eine Rückbewegung des Abstandshalters (13) in seine Ausgangsposition hervorruft.

9. Fußeinsatz nach einem der vorhergehenden Ansprüche, **dadurch gekennzeichnet, dass** die Fersenfeder eine C-Feder (2) ist, die durch Fersenbelastung etwas zusammengedrückt wird, wobei die sich dadurch einstellende Abstandsänderung der beiden Federschenkel (2a, 2b) voneinander die Fersenauslenkung definiert.

10. Fußeinsatz nach Anspruch 9, **dadurch gekennzeichnet, dass** die Adaptierungseinrichtung bzw. deren Antrieb in dem von der C-Feder (2) umschlossenen Raum angeordnet ist.

11. Fußeinsatz nach Anspruch 9 oder 10, **dadurch gekennzeichnet, dass** die C-Feder (2) mit ihrem oberen Schenkel (2a) mit dem hinteren Ende der Vorfußfeder (3; 21) verbunden ist.

12. Fußeinsatz nach Anspruch 1 oder 2, **gekennzeichnet durch** eine flach sinusförmig ausgebildete Fersenfeder (24), die mit ihrem vorderen Schenkelende (24a) im mittleren Längsbereich der Vorfußfeder (3) an deren Unterseite befestigt ist, wobei die Adaptierungseinrichtung bzw. deren Antrieb zwischen dem hinteren Schenkelende (24b) der Fersenfeder (24) und dem hinteren Ende der Vorfußfeder (3) angeordnet ist.

13. Fußeinsatz nach einem der vorhergehenden Ansprüche, **dadurch gekennzeichnet, dass** die Vorfußfeder (3; 21) aus zwei parallel geschalteten Blattfederelementen (9, 10) besteht, die mit ihrem hinteren Ende momentenstarr gekoppelt sind.

14. Fußeinsatz nach Anspruch 13, **dadurch gekennzeichnet, dass** die beiden parallel geschalteten Blattfederelemente (9, 10) in ihren beiden Endbereichen (A, B) momentenstarr gekoppelt sind.

15. Fußeinsatz nach Anspruch 1, 9 oder 10, **gekennzeichnet durch** eine als Basisfeder (21) ausgebildete Vorfußfeder und **durch** eine im Fersenbereich angeordnete C-Feder (2), die mit ihrem oberen C-Schenkel (2a) die Prothesenbelastung aufnimmt und mit ihrem unteren C-Schenkel (2b) auf einem von dem hinteren Ende der Basisfeder (21) gebildeten, nach hinten hochgezogenen Sattel (25) aufliegt, der sich auf einem Fersenkeil (26) abstützt, wobei zwischen dem oberen C-Schenkel (2a) und einem hinter ihm angeordneten Lagerbock (27) eine Fesselung in Form eines Pneumatikzylinders (28) vorgesehen ist, der eine progressive Kraft-Weg-Kennlinie aufweist und an den Druckstutzen (12a) einer Luftpumpe (12) angeschlossen ist, die ebenfalls zwischen dem oberen C-Schenkel (2a) und dem Lagerbock (27) angelenkt ist.

## Claims

1. Resilient foot insert for an artificial foot, with a forefoot spring (3; 9, 10; 21) designed as a leaf spring, and with a heel spring (2; 24) which is arranged in the heel region and is likewise designed as a leaf spring, at least one of these two spring elements determining the spring rigidity of the artificial foot, and with an adapting device for changing the spring rigidity of the at least one spring element determining the spring rigidity of the artificial foot, **characterized in that** the adapting device is actuated by a heel deflection in the foot insert effected as a function of the respective loading to which the foot insert is subjected by the patient wearing the artificial foot.

2. Foot insert according to Claim 1, **characterized in that** the spring element to be changed in terms of its spring rigidity is the forefoot spring (3) which extends over almost the entire length of the foot insert, and **in that** the heel deflection in the foot insert, being adjusted by the heel loading, serves as input variable for a forced regulation of the adapting device.

3. Foot insert according to Claim 2, **characterized in that** the forefoot spring (3) is acted upon by an air-filled pressure pad (11) which determines its spring rigidity, and the adapting device is an air pump (12) which is driven by the heel deflection and thus, with increasing heel loading, increases the air pressure in the pressure pad (11), while, with decreasing heel loading, the air pressure in the pressure pad (11) decreases.

4. Foot insert according to Claim 3, **characterized in that** the pressure pad (11) is assigned a positive-pressure valve which forms a constant positive-pressure leakage for reducing the air pressure within a predetermined period of time.

5. Foot insert according to Claim 3, **characterized in that** the adapting device comprises an intermediate store which memorizes the current value of the heel deflection, it being the case that with increasing heel deflection further air is pumped into the pressure pad (11), but with decreasing heel deflection the air pressure in the pressure pad (11) is reduced.

6. Foot insert according to Claim 3, 4 or 5, **characterized in that** the air-filled pressure pad (11) is assigned a contoured abutment surface (23) whose surface (C), which is projected onto the pressure pad (11), upon penetration into the pressure pad (11), is adapted to the progression of the pressure increase, as a result of the decrease in volume in the pressure pad (11), such that at least approximately linear force/travel characteristics of the pressure pad (11) are obtained.

7. Foot insert according to one of Claims 3 to 6, **characterized in that** the air-filled pressure pad (11) rests on a forefoot spring (21) which is pivotable and extends into the forefoot and whose top side is acted upon by a rigidly designed branch (22) of the foot insert.

8. Foot insert according to Claim 1 or 2, **characterized in that** the adapting device is a rigid spacer (13) which can be displaced in the longitudinal direction with respect to the forefoot spring (3; 21), determines the spring rigidity of the latter and is displaced longitudinally by a mechanical adjustment device (14, 15, 16, 17; 19, 20) which is connected kinematically to the heel deflection, which, upon maximum deflection, causes maximum displacement of the spacer (13) in the direction of the front end of the forefoot spring (3; 21), while relief of pressure from the forefoot causes the spacer (13) to move back into its starting position.

9. Foot insert according to one of the preceding claims, **characterized in that** the heel spring is a C-spring (2) which is compressed somewhat under heel loading, the resulting change in distance between the two spring branches (2a, 2b) defining the heel deflection.

10. Foot insert according to Claim 9, **characterized in that** the adapting device or its drive is arranged in the space enclosed by the C-spring (2).

11. Foot insert according to Claim 9 or 10, **characterized in that** the C-spring (2) is connected, via its upper branch (2a), to the rear end of the forefoot spring (3; 21).

12. Foot insert according to Claim 1 or 2, **characterized by** a heel spring (24) which is designed in the form of a shallow sine wave and is secured, by way of its front branch end (24a), on the underside of the forefoot spring (3), in the central longitudinal region of the latter, it being the case that the adapting device or its drive is arranged between the rear branch end (24b) of the heel spring (24) and the rear end of the forefoot spring (3).

13. Foot insert according to one of the preceding claims, **characterized in that** the forefoot spring (3; 21) consists of two leaf-spring elements (9, 10) which are connected in parallel and which are coupled in a rigid manner with respect to moments at their rear end.

14. Foot insert according to Claim 13, **characterized in that** the two leaf-spring elements (9, 10) connected in parallel are coupled in their two end regions (A, B) in a rigid manner with respect to moments.

15. Foot insert according to Claim 1, 9 or 10, **characterized by** a forefoot spring designed as a base spring (21) and by a C-spring (2) which is arranged in the heel region, takes up the prosthesis loading via its upper C-branch (2a), and bears with its bottom C-branch (2b) on a rearwardly extended saddle (25) formed by the rear end of the base spring (21), which saddle is supported on a heel wedge (26), and, provided between the upper C-branch (2a) and a bearing block (27) which is arranged behind said branch, there is a tie in the form of a pneumatic cylinder (28) which has a progressive force/travel characteristic line and is connected to the pressure connection stub (12a) of an air pump (12) likewise articulated between the upper C-branch (2a) and the bearing block (27).

## Revendications

1. Insert de pied élastique pour un pied artificiel, comprenant un ressort d'avant-pied (3; 9,10 ;21) formé en tant que ressort à lame, et un ressort de talon (2; 24) agencé dans la région du talon, formé également en tant que ressort à lame, l'un au moins de ces deux éléments élastiques déterminant la raideur élastique du pied artificiel, l'insert comprenant en outre un dispositif d'adaptation pour modifier la raideur élastique de l'au moins un élément élastique déterminant la raideur élastique du pied artificiel, **caractérisé** et en ce que la sollicitation du dispositif d'adaptation a lieu dans l'insert de pied par une déflexion du talon qui s'établit en fonction de la charge subie à chaque instant par l'insert de pied du fait du patient portant le pied artificiel.

2. Insert de pied selon la revendication 1, **caractérisé en ce que** l'élément élastique à modifier en ce qui concerne sa raideur élastique est le ressort d'avant-pied (3), lequel s'étend à peu près sur toute la longueur de l'insert de pied, et **en ce que** la déflexion du talon, qui s'établit dans l'insert de pied du fait de la charge du talon, sert de grandeur d'entrée pour une régulation forcée du dispositif d'adaptation.

3. Insert de pied selon la revendication 2, **caractérisé en ce que** le ressort d'avant-pied (3) est sollicité par un coussin presseur (11) rempli d'air déterminant la raideur élastique du ressort d'avant-pied, et le dispositif d'adaptation est une pompe à air (12) qui est actionnée par la déflexion du talon et qui élève ainsi la pression d'air dans le coussin presseur (11) lorsque la charge sur la talon augmente, alors que la pression d'air diminue dans le coussin presseur (11) lorsque la charge sur le talon diminue.

4. Insert de pied selon la revendication 3, **caractérisé par** une valve de surpression associée au coussin presseur (11) et engendrant une fuite constante de surpression pour éliminer la pression d'air sur une durée prédéterminée.

5. Insert de pied selon la revendication 3, **caractérisé en ce que** le dispositif adaptateur comprend un accumulateur intermédiaire qui mémorise la valeur actuelle de la déflexion du talon, davantage d'air étant pompé dans le coussin presseur (11) lorsque la déflexion du talon augmente, alors que la pression d'air (11) diminue dans le coussin presseur (11) lorsque la déflexion du talon diminue.

6. Insert de pied selon la revendication 3, 4 ou 5, **caractérisé en ce qu'**au coussin presseur (11) rempli d'air est associée une surface d'appui profilée (23) dont la surface (C) projetée sur le coussin presseur (11) lors de la pénétration dans le coussin presseur (11) est adaptée à l'allure de l'augmentation de pression en conséquence de la diminution de volume dans le coussin presseur (11) de telle manière qu'il en résulte une caractéristique force-course du coussin presseur (11) qui est au moins approximativement linéaire.

7. Insert de pied selon l'une des revendications 3 à 6, **caractérisé en ce que** le coussin presseur (11) rempli d'air repose sur un ressort d'avant-pied (11) pivotable s'étendant jusque dans l'avant-pied et est sollicité sur sa surface supérieure par une branche réalisée rigide (22) de l'insert de pied.

8. Insert de pied selon la revendication 1 ou 2, **caractérisé en ce que** le dispositif d'adaptation est une entretoise (13) rigide, déplaçable en direction longitudinale par rapport au ressort d'avant-pied (3; 21) et déterminant la raideur élastique de ce dernier, le déplacement longitudinal de l'entretoise ayant lieu par un dispositif de réglage mécanique (14, 15, 16, 17; 19, 20) qui est relié cinématiquement avec la déflexion du talon, laquelle a pour effet un déplacement maximal de l'entretoise (13) en direction de l'extrémité antérieure du ressort d'avant-pied (3; 21) en cas de déflexion maximale du talon, tandis qu'un déchargement de l'avant-pied induit un recul de l'entretoise (13) dans sa position de départ.

9. Insert de pied selon l'une des revendications précédentes, **caractérisé en ce que** le ressort de talon est un ressort en C (2) qui est un peu comprimé par la charge sur le talon, la modification de la distance qui s'établit ainsi entre les deux branches du ressort (2a, 2b) l'une de l'autre définissant la déflexion du talon.

10. Insert de pied selon la revendication 9, **caractérisé en ce que** le dispositif d'adaptation et en particulier son entraînement, est disposé dans l'espace entouré par le ressort en C (2).

11. Insert de pied selon la revendication 9 ou 10, **caractérisé en ce que** le ressort en C (2) est relié par sa branche supérieure (2a) avec l'extrémité arrière du ressort d'avant-pied (3; 21).

12. Insert de pied selon la revendication i ou 2, **caractérisé par** un ressort de talon (24) formé en sinusoïde plate, lequel est fixé par son extrémité antérieure (24a) dans la région longitudinale médiane du ressort d'avant-pied (3), sur le côté inférieur de celui-ci, le dispositif d'adaptation et en particulier son entraînement étant agencé entre l'extrémité arrière (24b) du ressort de talon (24) et l'extrémité arrière du ressort d'avant-pied (3).

13. Insert de pied selon l'une des revendications précédentes, **caractérisé en ce que** le ressort d'avant-pied (3; 21) est constitué de deux éléments (9, 10) de ressort à lame montés en parallèle, qui sont accouplés par leurs extrémités arrière d'une manière rigide aux moments .

14. Insert de pied selon la revendication 13, **caractérisé en ce que** les deux éléments (9, 10) de ressort à lame montés en parallèle sont accouplés d'une manière rigide aux moments en leur deux régions d'extrémité (A, B).

15. Insert de pied selon la revendication 1, 9 ou 10, **caractérisé par** un ressort d'avant-pied formé en tant que ressort de base (21), et par un ressort en C (2) disposé dans la région du talon, et qui supporte la charge de la prothèse par la branche supérieure (2a) du C et repose par sa branche inférieure (2b) du C sur une selle (25) relevée vers l'arrière constituant l'extrémité arrière du ressort de base (21) et reposant sur une cale de talon (26), et il est prévu entre la branche supérieure (2a) du C et un bloc-support (27) disposé derrière cette branche supérieure un lien sous la forme d'un vérin pneumatique (28) qui présente une caractéristique force-course progressive, et qui est raccordé au raccord de pression (12a) d'une pompe à air (12), qui est également articulée entre la branche supérieure (2a) du C et le bloc support (27).
